# EUROPEAN PATENT APPLICATION

(11) **EP 0 719 778 A1**
(43) Date of publication of application: **03.07.1996**
(21) Application number: 96200597.1
(22) Date of filing: 22.03.1994
(51) Int. Cl.: C07D 503/00, A61K 31/42

(54) **Diamine salts of clavulanic acid**

(30) Priority: 26.03.1993 EP 93200872
(62) Divisional of application: 94911952.3
(71) Applicant: GIST-BROCADES N.V., NL-2600 MA Delft (NL)
(72) Inventor: Weber, Pieter G., NL-2985 BN Ridderkerk (NL)
(74) Representative: Visser-Luirink, Gesina, Dr.

(57) **Abstract**

New diamine salts of clavulanic acid, pharmaceutical compositions comprising the same, and a new process using these diamine salts in the preparation of clavulanic acid and salts and esters thereof have been provided.

## Description

The present invention relates to new diamine salts of clavulanic acid, pharmaceutical compositions thereof, and to the use of these salts in the production of clavulanic acid and salts and esters thereof.

GB patent 1508977 discloses that clavulanic acid, which has the formula (I): 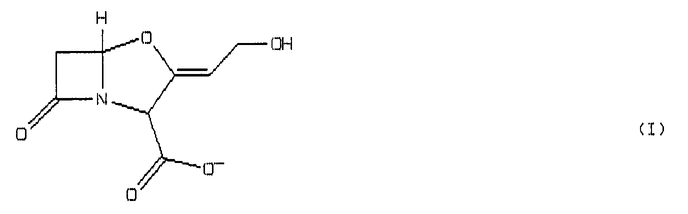
and its pharmaceutically acceptable salts and esters are anti-bacterial agents, able to enhance the effectiveness of penicillins and cephalosporins against many β-lactamase-producing bacteria.

US patent 4,650,795 discloses a group of primary amine salts of clavulanic acid which give stable pharmaceutical compositions.

EP patent 26044 discloses the use of the t-butylamine salt as a useful intermediate in the preparation of clavulanic acid.

The non-prepublished EP patent application 562583 discloses the same use for N,N-diisopropylethylenediammonium di clavulanate and N,N-diethylethylenediammonium di clavulanate, both of the secondary, secondary type.

Surprisingly it has been found that tertiary, tertiary diamine salts of clavulanic acid have improved properties compared to the t-butyl amine salt of clavulanic acid mentioned above.

Accordingly, the present invention provides tertiary, tertiary diamine di salts of formula (IIb): 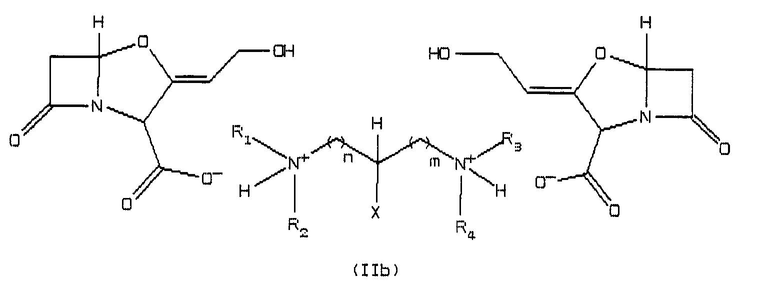
where R₁ and R₂ are each a (1-8C)alkyl, (3-8C)cycloalkyl or (3-8C)cycloalkyl(1-8C)alkyl group, optionally having one or more inert substituents or are interlinked to form a ring of 4-7 ring atoms; R₃ and R₄ are each a (1-8C)alkyl, (3-8C)cycloalkyl or (3-8C)cycloalkyl(1-8C)alkyl group, optionally having one or more inert substituents or are interlinked to form a ring of 4-7 ring atoms; X is hydrogen or a hydrogen bridge forming group; and m and n are each, independently, 0-5.

An alkyl group may be branched or straight chain.

A C₁ to C₈ alkyl group is preferably a C₁ to C₄ alkyl group, for example, methyl, ethyl, propyl, isopropyl, butyl, sec.butyl or tert.butyl, more preferably it is methyl.

A C₃ to C₈ cycloalkyl group is preferably a C₅ to C₇ cycloalkyl group, for example cyclopentyl, cyclohexyl or cycloheptyl.

Preferably, X is hydrogen, hydroxy or halogen, for instance bromine or chlorine. Most preferably, X is hydrogen or hydroxy.

Preferably n is from 0 to 3 and m is from 0 to 3, more preferably when X is hydrogen or hydroxy. Most preferably n = 1, m is 0 and X is hydrogen or n is 1, m is 1 and X is hydroxy.

Suitably inert substituents include halogen, hydroxy, C₁ to C₄ alkyl, C₁ to C₄ alkoxyl, C₁ to C₄ acyloxyl and C₁ to C₄ esterified carboxyl.

A halogen atom is, for example, bromine, chlorine or fluorine, preferably, bromine or chlorine.

A C₁ to C₄ alkyl is preferably methyl or ethyl.

A C₁ to C₄ alkoxyl is preferably methoxyl or ethoxyl.

A C₁ to C₄ acyloxyl is preferably C₁ or C₂ acyloxyl.

A C₁ to C₄ esterified carboxyl is preferably C₁ or C₂ esterified carboxyl.

Generally, R₁, R₂, R₃ and R₄ have three substituents or fewer, preferably two substituents or fewer. Most preferably R₁, R₂, R₃ and R₄ have one substituent or are unsubstituted.

When R₁ and R₂ or R₃ and R₄ are interlinked to form a ring of 4 to 7 atoms, the ring consists preferably of carbon atoms and is most preferably saturated. Most preferably R₁, R₂, R₃ and R₄ are methyl.

Normally the amine of the formula (III): 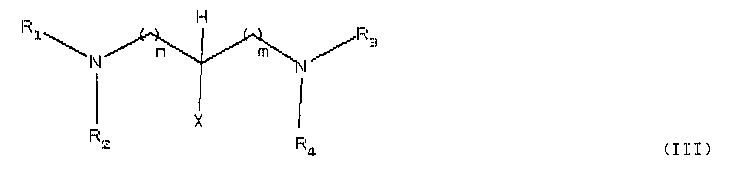
from which the salts of the formula (IIb) are derivable is a pharmaceutically acceptable amine.

Preferably, the salts of the formula (IIb) are derivable from N,N,N',N'-tetramethyl-1,2-diaminoethane, 1,3-bis(dimethyl-amino)-2-propanol, N,N,N',N'-tetramethyl-1,4-diaminobutane, N,N,N',N'-tetramethyl-1,6-diaminohexane, 1,2-dipiperidinoethane and dipiperidinomethane.

The present invention also provides a process for the preparation of a salt of formula (IIb) which process comprises the reaction of clavulanic acid with diamine (III): 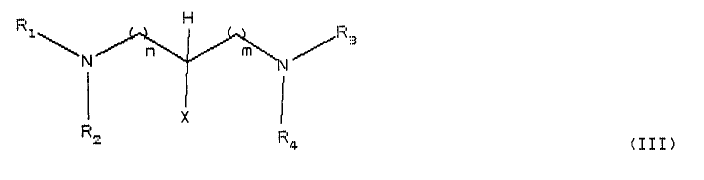
where R₁, R₂, R₃, R₄, X, m and n are as above defined. A diamine mono clavulanate will be formed when the amount of diamine is relatively high compared to that of clavulanic acid and the diamine di clavulanate will be formed when the amount of diamine is relatively low compared to that of clavulanic acid or a mixture of the same at a concentration in between.

The conditions when mono or di salts of clavulanic acid or a mixture of the same will be formed have not been investigated for each diamine, but it will be clear for someone skilled in the art that these will vary with the diamine applied.

The concentration of diamine present in the reaction mixture may be varied by, for example, varying the pH. At relatively high pH (dependent on the amine and the solvent used) more mono-protonated diamine (IIIa): 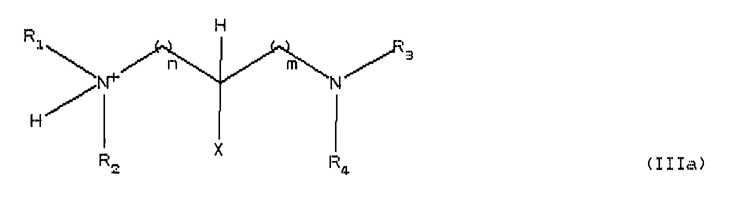
will be present and therefore more mono salt will be precipitated, and at relatively low pH more di-protonated diamine (IIIb): 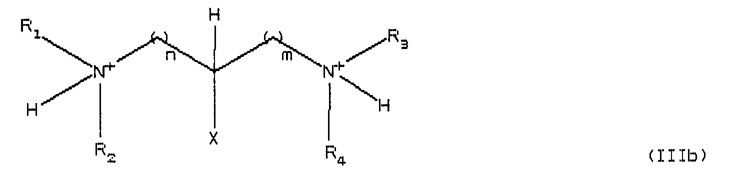
will be present and therefore more di salt will be precipitated.

The present invention further provides the use of diamine salts of clavulanic acid as defined above as an intermediate in the preparation of clavulanic acid and a pharmaceutically acceptable salt or ester thereof.

In another aspect the present invention provides a process for the preparation of clavulanic acid or a pharmaceutically acceptable salt or ester thereof which process comprises converting a diamine salt of clavulanic acid as defined above into clavulanic acid, generally by acidification, or a pharmaceutically acceptable salt or ester thereof, generally by adding a source of corresponding salt or ester forming compound.

In a further aspect the present invention provides a process for the purification of clavulanic acid or a pharmaceutically acceptable salt or ester thereof which process comprises:
i) contacting impure clavulanic acid in an organic solvent with diamine to form a salt of formula (IIb);
ii) isolating the salt produced in step i); and
iii) converting the isolated salt into clavulanic acid, generally by acidification, or a pharmaceutically acceptable salt or ester thereof, generally by adding a source of a corresponding salt or ester forming compound. For instance, potassium clavulanate is formed by adding potassium acetate or potassium ethylhexanoate.

Most suitably the formation of the diamine salts of clavulanic acid takes place in an organic solvent. Suitable solvents include non-hydroxylic solvents such as, for example, tetrahydrofuran, dioxane, ethyl acetate, methyl acetate, acetone, methylethylketone and the like solvent and mixtures thereof

The reaction may be carried out at from about -50°C to 40°C, most preferably from about 0°C to 15°C.

The present invention also provides pharmaceutical compositions which comprise a salt of the formula (IIb) and a pharmaceutically acceptable carrier.

Suitable forms of the compositions of this invention include tablets, capsules, reconstitutable powders and sterile forms suitable for injection or infusion. Such compositions may contain conventional pharmaceutically acceptable materials such as diluents, binders, colours, flavours, preservatives and disintegrants.

Injectable or infusible compositions of the salts of formula (IIb) are particularly suitable as high tissue levels of the compound of clavulanic acid can occur after administration by injection or infusion. Thus, one preferred composition aspect of this invention comprises salts of the formula (IIb) in sterile form.

Unit dose compositions comprising a salt of the formula (II) adapted for oral administration form a further preferred composition aspect of this invention.

The following examples will illustrate the invention. The pH value mentioned relates to this value measured with an Ingold electrode, type U402-S7/120, in the solvents applied.

### Examples

### Example 1

### Comparison of crystallization of various diamine salts

A solution of potassium clavulanate in ice cold water was stirred with ethyl acetate under cooling with ice-water. With a solution of about 10% (w/w) sulphuric acid the pH was brought at about 2, the water layer separated and twice extracted with ethyl acetate. The collected extracts were dried with magnesium sulphate, filtered and washed with ethyl acetate, yielding a solution of clavulanic acid of about 2% (w/w). The clavulanic acid was diluted with an equal volume of acetone and diamine was added.

The results are summarized in Table I. The ratio moles diamine/moles clavulanic acid has been indicated in the last column. The tertiary, tertiary type diamine salts of clavulanic acid are present in the preferred crystal form only, viz. not in the form of an oil.

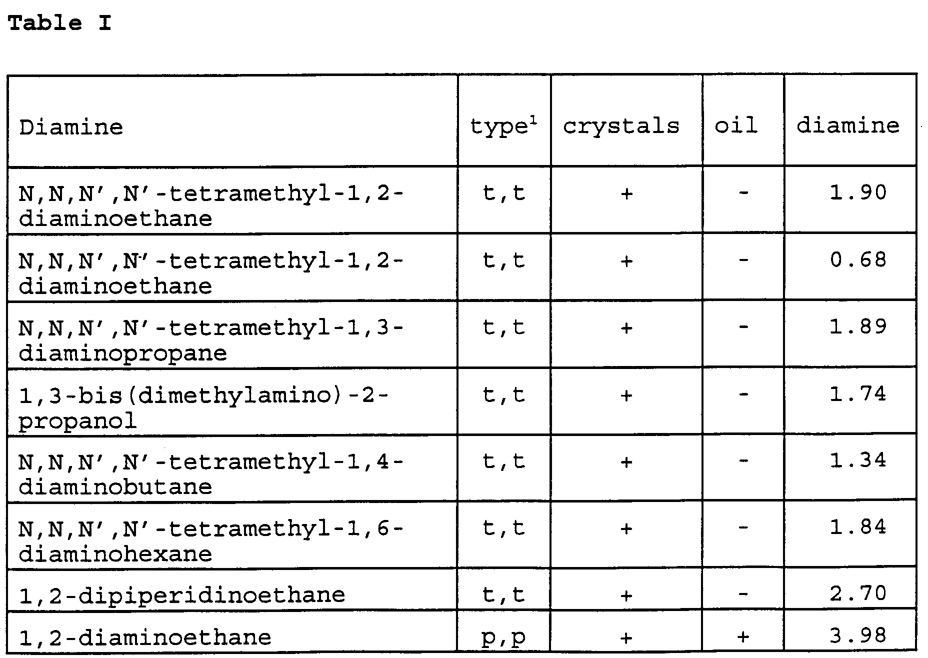

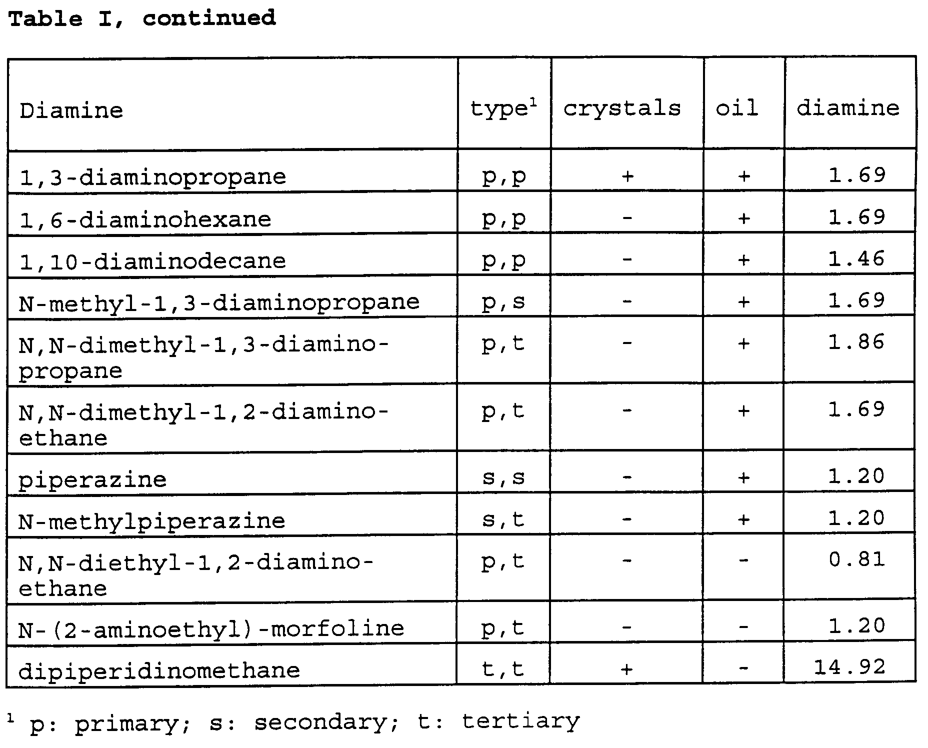

### Example 2

### Preparation of N,N,N',N'-tetramethyl-1,2-diaminoethane di clavulanate from ethyl acetate/acetone solution

A solution of clavulanic acid in ethyl acetate (200 ml containing 1.1 g of clavulanic acid) was added in 10 min to 200 ml of cold acetone (10°C) while stirring and keeping the pH between 7.5 and 8 with N,N,N',N'-tetramethyl-1,2-diaminoethane (TMEDA, 0.64 g, viz. a molar ratio of 1.45 related to clavulanic acid). Stirring was continued for 0.5 hr and the precipitate was filtered off, washed with acetone and dried in vacuum at 35°C to give 1.24 g of TMEDA di clavulanate (crystals in needle form). The mother liquor (337 g) contained 0.11 g of clavulanic acid.

NMR (DMSO-d6): 2.45 ppm, N-CH₃ (s, 12H); 2.80 ppm, N-CH₂ (s, 4H); 2.99 ppm, C6-βH (d, 2H); 3.53 ppm, C6-αH (dd, 2H); 3.99 ppm, CH₂OH (m, 4H); 4.69 ppm, =C-H (tr, 2H); 4.76 ppm, C3-H (s, 2H); 5.61 ppm, C5-H (d, 2H).

### Example 3

### Conversion of N,N,N',N'-tetramethyl-1,2-diaminoethane di clavulanate into potassium clavulanate

4.5 ml of a 2M solution of potassium 2-ethyl-hexanoate in isopropanol was added to a stirred solution of 2 g of N,N,N',N'-tetramethyl-1,2-diaminoethane di clavulanate (purity 75.8% calculated as the free acid) in 20 ml of isopropanol and 2 ml of water at room temperature. After stirring for 0.25 hr the precipitate was filtered off and washed with 5 ml of isopropanol. Drying in vacuum at room temperature gave 0.30 g of potassium clavulanate with a purity of 83.6% calculated as the free acid.

A further 2.5 ml of a 2M solution of potassium 2-ethylhexanoate in isopropanol was added to the mother liquor and stirring was continued. The precipitate was filtered off, washed with 5 ml of isopropanol and dried to give 0.68 g of potassium clavulanate with a purity of 82.3% calculated as the free acid.

## Claims

1. A salt of clavulanic acid of the formula (IIb): 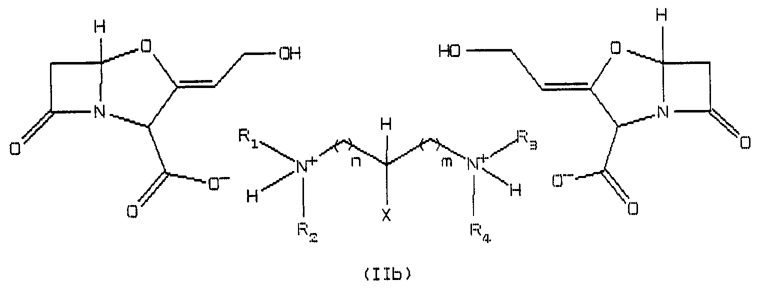 where R₁ and R₂ are each a (1-8C)alkyl, (3-8C)cycloalkyl or (3-8C)cycloalkyl(1-8C)alkyl group, optionally having one or more inert substituents or are interlinked to form a ring of 4-7 ring atoms; R₃ and R₄ are each a (1-8C)alkyl, (3-8C)cycloalkyl or (3-8C)cycloalkyl(1-8C)alkyl group, optionally having one or more inert substituents or are interlinked to form a ring of 4-7 ring atoms; X is hydrogen or a hydrogen bridge forming group; and m and n are each, independently, 0-5.

2. A salt of clavulanic acid according to claim 1 where R₁, R₂, R₃ and R₄ are methyl.

3. A salt of clavulanic acid according to claim 1 or 2 where X is hydrogen, n is 1 and m is 0.

4. A salt of clavulanic acid according to claim 1 or 2, wherein X is hydroxy, n is 1 and m is 1.

5. A salt of clavulanic acid of formula IIb according to claim 1, wherein R₁, R₂, R₃ and R₄ are methyl, X is hydrogen, n is 1 and m is 0.

6. A process for preparing a salt as claimed in claim 1, 2, 3 or 4, wherein clavulanic acid is reacted with a diamine (III): 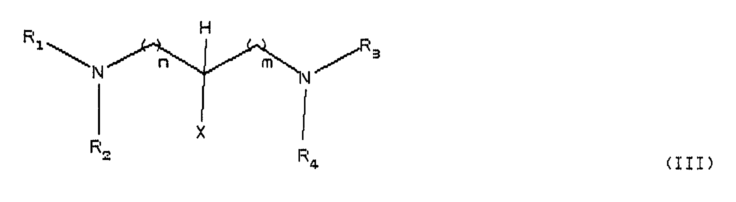 wherein R₁, R₂, R₃, R₄, X, m and n are as defined in claim 1, 2, 3 or 4, in an organic solvent.

7. A process according to claim 6 wherein the molar amount of diamine is less than that of clavulanic acid.

8. A process to prepare a salt as claimed in claim 5, wherein clavulanic acid is reacted with N,N,N',N'-tetramethyl-1,2-diaminoethane wherein the molar amount of N,N,N',N'-tetramethyl-1,2-diaminoethane is less than that of clavulanic acid.

9. A process for the preparation of clavulanic acid or a pharmaceutically acceptable salt or ester thereof which comprises converting a diamine salt as claimed in claim 1, 2, 3, 4 and 5 into clavulanic acid or a pharmaceutically acceptable salt or ester thereof.

10. A process for the purification of clavulanic acid or a pharmaceutically acceptable salt or ester thereof which process comprises:
i) containing impure clavulanic acid in an organic solvent with a diamine of formula III to form a salt of formula (IIb);
ii) isolating the salt produced in step i); and
iii) converting the isolated salt into clavulanic acid or a pharmaceutically acceptable salt or ester thereof.

11. A pharmaceutical composition comprising a salt of clavulanic acid as claimed in claim 1, 2, 3, 4 or 5 and a pharmaceutically acceptable carrier or diluent.

12. Use of a salt as claimed in claim 1, 2, 3, 4 or 5 as an intermediate in a process for the preparation of clavulanic acid or a pharmaceutically acceptable salt or ester thereof.
